# EUROPEAN PATENT APPLICATION

(11) **EP 3 205 664 A1**
(43) Date of publication of application: **16.08.2017**
(21) Application number: 16155165.0
(22) Date of filing: 11.02.2016
(51) Int. Cl.: C07K 14/605, C07K 1/10

(54) **METHOD FOR PREPARATION OF LIRAGLUTIDE USING BAL LINKER**

(71) Applicant: Polypeptide Laboratories Holding (PPL) AB, 200 61 Limhamn (SE)
(72) Inventor: The designation of the inventor has not yet been filed
(74) Representative: Malmqvist, Maria

(57) **Abstract**

The invention discloses a method for the preparation of liraglutide by SPPS using Backbone Amide Linker (BAL) and on resin introduction during SPPS of Palmitoyl-Glu-OtBu residue on the Lys⁽²⁰⁾.

SEQUENCE LISTING

<110>  Lonza Braine S.A.

<120>  Method for Preparation of Liraglutide using BAL linker

<130>  LP2434EP00

<160>  1

<170>  PatentIn version 3.5

<210>  1
<211>  31
<212>  PRT
<213>  Artificial

<220>
<223>  Synthetic construct

<400>  1

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Arg Gly Arg Gly
            20                  25                  30

## Description

The invention discloses a method for the preparation of liraglutide by SPPS using Backbone Amide Linker (BAL) and on resin introduction during SPPS of Palmitoyl-Glu-OtBu residue on the Lys⁽²⁰⁾.

### BACKGROUND OF THE INVENTION

Liraglutide has the CAS number 204656-20-2 and is compound of formula (3), 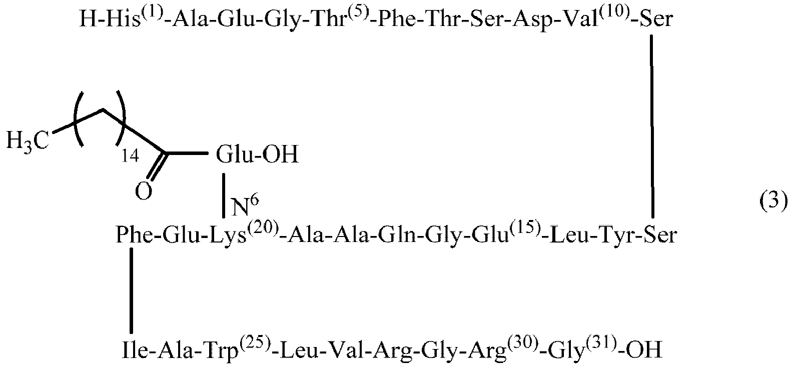 the sequence can also be described as follows:
H-His⁽¹⁾-Ala-Glu-Gly-Thr⁽⁵⁾-Phe-Thr-Ser-Asp-Val⁽¹⁰⁾-Ser-Ser-Tyr-Leu-Glu⁽¹⁵⁾-Gly-Gln-Ala-Ala-Lys(²⁰)(Palmitoyl-Glu)-Glu-Phe-Ile-Ala-Trp(²⁵)-Leu-Val-Arg-Gly-Arg(³⁰)-Gly(³¹)-OH;
the numbers in parenthesis show the numbering of the positions of the AA in the sequence as the numbering is used in instant invention.

WO 98/08871 A1 discloses a method for preparation of derivatives of GLP-1 and analogues thereof by culturing a host cell culture containing a DNA sequence encoding the polypeptide and capable of expressing the polypeptide in a suitable nutrient medium under conditions permitting the expression of the peptide, after which the resulting peptide is recovered from the culture.

WO 00/55119 A1 discloses the acylation of the epsilon-amino residue of Lys⁽²⁰⁾ of GLP-1 analogues with certain compounds useful as acylating agents. In this disclosure the N-terminal amino residue of GLP-1 analogues are not protected. The disadvantage is that diacylation occurs, both the N-terminal amino residue and the epsilon-amino residue of Lys⁽²⁰⁾ are acylated. The undesired by products need to be separated by purification.

Jensen et al., J. Am. Chem. Soc. 1998, 120, 5441-5452, discloses the use of Backbone Amide Linker (BAL) for SPPS of C-terminal-modified peptides.

There was a need for a method for preparation of liraglutide with high yields and that has not the disadvantage of producing by products by said diacylation.

A method was found that uses BAL and incorporates the Palmitoyl-Glu-OtBu residue on the Lys⁽²⁰⁾ during SPPS and on resin while the N-terminal NH₂ is still protected. If it was introduced onto a fully deprotected liraglutide precursor that has not yet the Palmitoyl-Glu-OtBu residue, a method that could be derived from the disclosure of WO 00/55119 A1, then the N-terminal NH₂ first needs to be selectively protected before the Palmitoyl-Glu-OtBu residue can be introduced onto the Lys⁽²⁰⁾ in order to avoid diacylation. This required selective protection is an additional difficulty, an additional source of impurities and introduces further steps in the synthesis of liraglutide.
Another advantage of the method is that the Palmitoyl-Glu-OtBu residue can be introduced with a good yield and with low side reactions by introducing it after the coupling of Ala⁽¹⁸⁾ or after the coupling of Ala⁽¹⁹⁾, thereby no or only low amounts of by products are formed, which would need to be separated.
The method provides for high yields and low impurity profiles.

In the following text,
AA amino acid;
Acm acetamidomethyl;
ACN acetonitrile;
Alloc allyloxycarbonyl protecting group;
BAL Backbone Amide Linker, also called BAL Linker;
Boc tert-butoxycarbonyl protecting group;
   coupling also called coupling reaction;
DCM dichloromethane;
DIC Diisopropylcarbodiimide;
DIPCDI Diisopropylcarbodiimide;
DMF Dimethylformamide;
Dnp dinitrophenyl;
eq equivalents;
Fmoc 9-fluorenylmethoxycarbonyl protecting group;
HFIP hexafluoroisopropanol;
MIS 1,2-dimethylindole-3-sulfonyl;
Mtr 4-methoxy-2,3,6-trimethylphenylsulfonyl;
N⁶ N⁶ denotes the NH₂ of the side chain of Lys;
N-terminal NH₂ N-terminal amino function;
OxymaPure® Ethyl (hydroxyimino)cyanoacetate, CAS 3849-21-6, purchased from Luxembourg Bio Technologies;
Pal, Palm abbreviations for Palmitoyl;
Pbf 2,2,4,6,7-pentamethyldihydrobenzofuran-5-sulfonyl protecting group;
Pmc 2,5,7,8-pentamethylchroman-6-sufonyl;
RT room or ambient temperature;
SPPS Solid Phase Peptide Synthesis;
tBu tert butyl or tBu protecting group;
OtBu tert butyl ester;
TFA trifluoroacetic acid;
TFE trifluoroethanol;
TIS triisopropylsilane;
Trt trityl;
Z benzyloxycarbonyl;
if not otherwise stated.

### SUMMARY OF THE INVENTION

Subject of the invention is a method for the preparation of liraglutide with SPPS using a backbone amide linker BAL for connecting the C-terminal Gly⁽³¹⁾ with the resin during SPPS;
BAL is compound of formula (BAL); 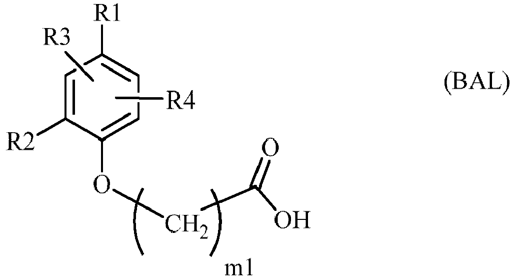 wherein
one of the substituents R1 and R2 is H and the other one is C(O)H;
ml is 1, 2, 3, 4, 5, 6, 7, 8, 9 or 10;
R3 and R4 are identical or different and independently from each other H or methoxy.

### DETAILED DESCRIPTION OF THE INVENTION

Preferably, ml is 2, 3, 4, 5, 6 or 7;
more preferably, ml is 3, 4, 5 or 7;
even more preferably, ml is 3 or 4;
even more preferably, ml is 3.

Preferably, R3 and R4 are on the 3 and on the 5 position of the phenoxy ring with respect to
the position of the carboxylic acid residue on position 1 of the phenoxy ring. Preferably, R2 is H and R1 is C(O)H.
More preferably, R3 and R4 are on the 3 and on the 5 position of the phenoxy ring with respect to the position of the carboxylic acid residue on position 1 of the phenoxy ring; and
R2 is H and R1 is C(O)H.

Even more preferably, R3 and R4 are on the 3 and on the 5 position of the phenoxy ring with respect to the position of the carboxylic acid residue on position 1 of the phenoxy ring; and
R2 is H and R1 is C(O)H; and
ml is 3.

Especially, BAL is compound of formula (BAL-1) which has CAS 197304-21-5.

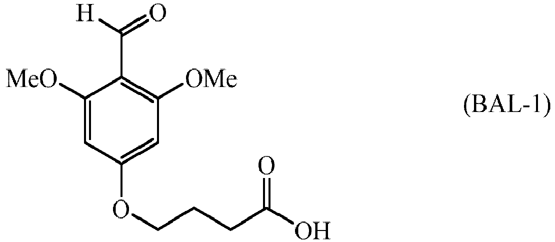

Preferably, the SPPS is done on an amino functionalized resin AMFUNRESIN.
In principle AMFUNRESIN can be any amino functionalized resin conventionally used in SPPS, since like in whatever SPPS, the structure of the resin and its characteristics (such as substitution,granulometry, percentage of reticulation, and so on) of the resin will influence only the swelling of the resin, but will have no direct implication on the SPPS.
Preferably, AMFUNRESIN is selected from the group consisting of aminomethyl ChemMatrix® resin, aminomethyl polystyrene resin, Rink amide resin, Sieber resin, 4-methyl-benzhydrylamine resin (MBHA resin), poly Lys resin, and benzhydrylamine resin (BHA resin).
Preferably, AMFUNRESIN is an aminomethyl ChemMatrix® resin, Rink amide resin or Sieber resin.

Preferably, the resin used in the SPPS shows a loading capacity of from 0.5 to 2 mmol of reactive sites per g resin.

Preferably, BAL is covalently connected with the resin by an amide bond between the COOH of BAL and the NH₂ of AMFUNRESIN.

Preferably, Gly⁽³¹⁾ is covalently connected with BAL by a bond between the alpha NH₂ of Gly⁽³¹⁾ and the C atom of the C(O)H residue of BAL.
Preferably, Gly⁽³¹⁾ is used with its alpha COOH in protected form, preferably in the form of H-Gly-OtBu, that is with an tBu ester on the alpha COOH.

Preferably, liraglutide is prepared by SPPS by firstly covalently connecting the BAL with AMFUNRESIN. Thereafter the alpha NH₂ of Gly⁽³¹⁾ is covalently connected with the C(O)H residue of BAL. Then the other AA are coupled by SPPS onto the Gly⁽³¹⁾ and then onto the growing peptide chain respectively.

The covalently connecting of BAL with AMFUNRESIN is done in a reaction REACBONBALRES; REACBONBALRES is a coupling reaction and can be done with the reagents and the conditions as described for COUPL.
Preferably, the molar amount of BAL used in REACBONBALRES is from 1 to 10 times, more preferably from 1 to 8 times, even more preferably from 2 to 7 times, of the molar loading capacity of the resin.

Preferably, the covalently connecting of the alpha NH₂ of Gly⁽³¹⁾ with the C(O)H residue of BAL is done with a reductive amination reaction, which comprises two consecutive reactions, a reaction REACONBALGLY1, which is a condensation reaction, and a reaction REACBALGLY2, which is a reduction.
Preferably, REACONBALGLY1 and REACBALGLY2 are done in a solvent.
Preferably, REACONBALGLY1 is done in the presence of an acid, the acid is preferably AcOH.
Preferably, the volume of acid is from 50 to 250 micorL per g of resin, more preferably from 75 to 150 microL per g of resin.
Preferably, the acid is used in a mixture of from 0.1 to 10%, preferably from 0.2 to 5%, more preferably from 0.2 to 3%, of acid with the solvent, the % are vol-% and are being based on the total volume of the mixture of acid and solvent.
REACBALGLY2 is preferably done with a reducing agent, the reducing agent is preferably NaBH₃CN.
Preferably, the molar amount of reducing agent is from 1 to 20 times, more preferably from 1 to 15 times, of the molar loading capacity of the resin.
Preferably, the reducing agent is used in a mixture of from 0.1 to 10%, preferably from 0.2 to 5%, more preferably from 0.2 to 3%, of reducing agent with a solvent, the % are vol-% and are being based on the total volume of the mixture of reducing agent and solvent.
The solvent for the two reactions and for the reducing agent can be any solvent that is inert against the reducing agent used in the reduction, preferably, the solvent is DMF, MeOH, THF, MeTHF, NMP or a mixture thereof; more preferably DMF, MeOH or a mixture thereof.
Preferably, REACONBALGLY1 and REACBALGLY2 are done at atmospheric pressure.
Preferably, the reaction temperature of REACONBALGLY1 and of REACBALGLY2 respectively is from -20 to 70°C; more preferably from -15 and 40°C, even more preferably from -15 to 35°C.

Preferably, the reaction time of REACONBALGLY1 and of REACBALGLY2 respectively is from 10 min to 48 h, more preferably from 10 min to 24 h, even more preferably from 10 min to 12 h.

Preferably, the method comprises two steps, a step STEP 1 and a step STEP2;
STEP 1 comprises the SPPS using a backbone amide linker BAL;
STEP1 provides liraglutide connected to the resin of the SPPS;
STEP2 comprises cleavage of liraglutide from the resin.

Preferably, those AA, that can be side chain protected, are used in a side chain protected form in the SPPS.
Preferably, STEP1 provides a protected liraglutide connected to the resin of the SPPS.
Preferably, STEP2 comprises deprotection of the protected liraglutide.
Preferably, STEP2 provides liraglutide.

Preferably, the side chain protection is realized in form of side chain protection groups which are not cleavable under weakly acidic conditions, with the weakly acidic conditions as described herein.

Preferably, side chain protection of Cys is Trt or Acm.
Preferably, side chain protection of Asn and Gln is Trt.
Preferably, side chain protection of His is Trt or Dnp.
Preferably, side chain protection of Glu and Asp is OtBu.
Preferably, side chain protection of Thr, Ser and Tyr is tBu.
Preferably, side chain protection of Trp is Boc.
Preferably, side chain protection of Arg is Pbf, Pmc, Mtr or MIS.
Preferably, side chain protection of Lys is Alloc, Z or Boc.

Preferably, the side chain protection is realized in form of side chain protection groups which are cleavable under strongly acidic conditions, with the strongly acidic conditions as described herein;
more preferably except for Lys, where the side chain protection in Alloc, Z or Boc.

More preferably, side chain protection of Cys, Asn, His and Gln is Trt;
side chain protection of Glu and Asp is OtBu;
side chain protection of Thr, Ser and Tyr is tBu;
side chain protection of Trp is Boc;
side chain protection of Arg is Pbf;
side chain protection of Lys is Alloc.

Preferably, the SPPS is done by stepwise coupling of the individual amino acids,
more preferably except for the Val(¹⁰) and the Ser⁽¹¹⁾; the Val(¹⁰) and the Ser⁽¹¹⁾ are preferably used in the SPPS in form of pseudoproline Fmoc-Val-Ser[Psi^{(Me,Me)}Pro]-OH.

Preferably, the SPPS uses Fmoc/tBu strategy; with Fmoc as protecting group for the alpha NH₂ of the amino acids used in the SPPS;
more preferably except for the N-terminal His⁽¹⁾, where the alpha NH₂ is protected with Boc.

Cleavage of the peptide from the resin, of any side chain protecting group, of any protecting group on the N-terminal NH₂, of any protecting group on the C-terminal COOH, also the cleavage of the tBu protecting group on the alpha COOH of the Glu of the Palmitoyl-Glu-OtBu residue, can be done separately or simultaneously. The cleavage is preferably done in STEP2. When the cleavage is done simultaneously, then the cleavage is usually called global deprotection.
The conditions of such cleavage reactions are known to the skilled person and are dependent on the nature of protecting group. Many protecting groups require acidic conditions for cleavage, preferably strongly acidic conditions, with the strongly acidic conditions as defined herein, other protecting groups such as Z require cleavage by catalytic hydrogenation.
Such cleavage reaction or such cleavage reactions can be and is preferably done under strongly acidic conditions, more preferably the cleavage is done in form of a global deprotection.

Strongly acidic conditions means, in the context if this invention, using for cleavage a mixture of from 50 to 100%, preferably from 60 to 100%, more preferably from 70 to 100%, even more preferably from 80 to 100%, of TFA with a component COMPSTRONG, the % are vol-% and are being based on the total volume of the mixture of TFA and COMPSTRONG.

Therefore, TFA can also be used neat.
Preferably, COMPSTRONG is selected from the group consisting of SOLVSTRONG, phenol, water, TIS and a mixture thereof;
SOLVSTRONG is solvent that is inert against TFA.
Preferably, SOLVSTRONG is DCM.
more preferably, COMPSTRONG is a mixture of phenol and TIS or a mixture of phenol, water and TIS.
Preferably, the volume ratios of a mixture TFA : water : TIS are
TFA: from 80 to 98;
water: from 1 to 10;
TIS: from 1 to 10;
with the individual volume ratios of the three components adding up to 100.
Preferably, the volume ratios of a mixture TFA : phenol: water : TIS are
TFA: from 70 to 97;
phenol: from 1 to 10;
water: from 1 to 10;
TIS: from 1 to 10;
with the individual volume ratios of the four components adding up to 100.
Preferably, the total amount of the mixture is from 5 to 20 ml per g of protected peptide.
Preferably, the cleavage is done at atmospheric pressure.
Preferably, the reaction temperature of the cleavage is from -20 to 70°C; more preferably from -15 and 40°C, even more preferably from -15 to 35°C.
Preferably, the reaction time of the cleavage is from 30 min to 12 h, more preferably from 30 min to 6 h, even more preferably from 30 min to 4 h.

Weakly acidic conditions means, in the context if this invention, using for cleavage a mixture of from 0.01 to 25%, preferably from 0.01 to 15%, more preferably from 0.05 to 10%, even more preferably from 0.1 to 7.5%, TFA in a solvent SOLVWEAK, the % are vol-% and are being based on the total volume of the mixture of TFA and SOLVWEAK.
SOLVWEAK is any solvent which is inert against TFA.
Preferably SOLVWEAK is DCM, methyl THF or a mixture thereof.
Preferably, the total amount of the mixture is from 5 to 20 ml per g of protected peptide.
Preferably, the cleavage is done at atmospheric pressure.

Preferably, the reaction temperature of the cleavage is from -20 to 70°C; more preferably from -15 and 40°C, even more preferably from -15 to 35°C.
Preferably, the reaction time of the cleavage is from 5 min to 12 h, more preferably from 5 min to 6 h, even more preferably from 10 min to 4 h.

Cleavage under weakly acidic conditions can also be done using TFE or HFIP instead of TFA.

Preferably, the Palmitoyl-Glu-OtBu residue on the N⁶ of the Lys⁽²⁰⁾ is the residue of formula (PALGLU), 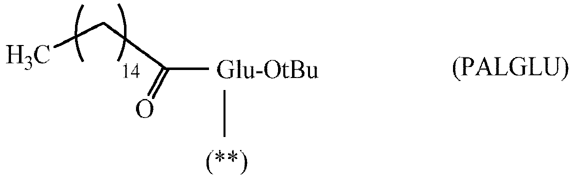
with the (**) in formula (PALGLU) denoting the covalent bond between the COOH of the side chain of the Glu and the N⁶ of the Lyes⁽²⁰⁾,
and the Palmitoyl-Glu-OtBu residue is covalently bonded to the Lys⁽²⁰⁾ in a reaction REACPALGLU, wherein the NH₂ of the side chain of the Lys is reacted with a precursor of the residue of formula (PALGLU);
preferably, REACPALGLU is done before the peptide is cleaved from the resin.

So REACPALGLU is done on-resin, that is REACPALGLU is done while the peptide is still covalently bonded to the resin.

Preferably, the precursor of the residue of formula (PALGLU) is Pal-Glu(OSu)-OtBu, that is compound of formula (10); 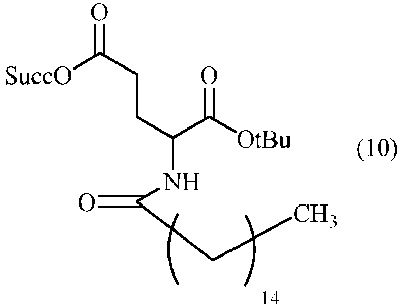 with SuccO being residue of formula (SuccO); 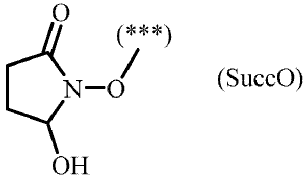 wherein the (***) denotes the covalent bond to the CO residue in compound of formula (10).

Preferably, REACPALGLU is done after the coupling of Ala⁽¹⁹⁾ or after the coupling of Ala⁽¹⁸⁾, and before removal of the protecting group of the alpha NH₂ of Ala⁽¹⁹⁾ or of Ala⁽¹⁸⁾ respectively, preferably after the coupling of Ala⁽¹⁸⁾.
Preferably, any protecting group of the side chain of Lys⁽²⁰⁾ is cleaved before REACPALGLU.

Preferably, Lys⁽²⁰⁾ is used in the SPPS in form of Fmoc-Lys(Alloc)-OH;
so the side chain protecting group of Lys⁽²⁰⁾, which is cleaved before REACPALGLU, is preferably Alloc.
Preferably, cleavage of Alloc from the Lys⁽²⁰⁾ is done with a reaction CLEAVALLOC using Pd(PPh₃)₄ and PhSiH.
CLEAVALLOC can be done in the presence of an additive ADDALLOC, ADDALLOC is selected from the group consisting of morpholine, 1,3-dimethylbarbituric acid, pyrrolidine, triphenylphosphine, dimedone, and mixtures thereof.
Preferably, the molar amount of ADDALLOC is from 2 to 7 times of the molar amount of the Alloc residue.

Preferably, the molar amount of Pd(PPh₃)₄ is from 0.01 to 10 times, more preferably from 0.02 to 5 times, even more preferably from 0.05 to 2 times, of the molar loading capacity of the resin.
Preferably, the molar amount of PhSiH is from 1 to 30 times, more preferably from 2 to 20 times, even more preferably from 5 to 15 times, of the molar loading capacity of the resin.
Preferably, CLEAVALLOC is done in a solvent selected from the group consisting of DMSO, dichloromethane, Me-THF, DMF, NMP, and mixtures thereof, more preferably the solvent is DCM.
Preferably, CLEAVALLOC is done at atmospheric pressure.
Preferably, the reaction temperature of CLEAVALLOC is from -20 to 70°C; more preferably from -15 and 60°C, even more preferably from 5 to 35°C.
Preferably, the reaction time of CLEAVALLOC is from 1 min to 2 h.

Preferably, the molar amount of Pal-Glu(OSu)-OtBu in REACPALGLU is from 1 to 15 times, more preferably from 1 to 10 times, even more preferably from 1 to 5 times, of the molar loading capacity of the resin.
REACPALGLU can be done in the presence of a base, preferably a tertiary amine base.
Suitable bases are for example trialkylamines, like N,N-diisopropylethylamine (DIPEA) or triethylamine (TEA);
N,N-dialkylanilines, like N,N-diethylaniline;
2,4,6-trialkylpyridines, like 2,4,6-trimethylpyridine; and
N-alkylmorpholines, like N-methylmorpholine.
Preferably, REACPALGLU is done in the presence of DIPEA as a base.
Preferably, the molar amount of base is from 1 to 10 times, more preferably from 3 to 8 times, of the molar loading capacity of the resin.
Preferably, REACPALGLU is done in a solvent selected from the group consisting of DMSO, dichloromethane, Me-THF, DMF, NMP, and mixtures thereof, more preferably the solvent is DCM.
Preferably, REACPALGLU is done at atmospheric pressure.
Preferably, the reaction temperature of REACPALGLU is from -20 to 70°C; more preferably from -15 and 60°C, even more preferably from 5 to 35°C.
Preferably, the reaction time of REACPALGLU is from 1 to 48 h, more preferably from 5 h to 24 h, even more preferably from 10 h to 24 h.

Preferably, any coupling, which for the ease of reading is also called COUPL, such as coupling during SPPS or REACBONBALRES, can be carried out using reaction conditions known in the art of peptide synthesis.
Preferably, coupling reagents, which can be used for COUPL and which are used in situ, are for example phoshonium or uronium coupling reagents, like benzotriazol-1-yloxytris(dimethylamino)phosphonium hexafluorophosphate (BOP), benzotriazol-1-yloxytris(pyrrolidino )phosphonium hexafluorophosphate (PyBOP), O-(benzotriazol-1-yl)-1,1,3,3-tetramethyluronium hexafluorophosphate (HBTU), O-(6-chlorobenzotriazol-1-yl)-1,1,3,3-tetramethyluronium hexafluorophosphate (HCTU), O-(6-chlorobenzotriazol-1-yl)-1,1,3,3-tetramethyluronium tetrafluoroborate (TCTU), O-(7-azabenzotriazol-1-yl)-1,1,3,3-tetramethyluronium hexafluorophosphate (HATU), O-(7-azabenzotriazol-1-yl)-1,1,3,3-tetramethyluronium tetrafluoroborate (TATU), O-(benzotriazol-l-yl)-1,1,3,3-tetramethyluronium tetrafluoroborate (TBTU), O-[cyano(ethoxycarbonyl)methylenamino]-1,1,3,3-tetramethyluronium tetrafluoroborate (TOTU) and (1-cyano-2-ethoxy-2-oxoethylidenaminooxy)dimethylamino-morpholino-carbenium hexafluorophosphate (COMU);
or carbodiimide coupling reagents, like diisopropylcarbodiimide (DIC), dicyclohexylcarbodiimide (DCC) and water-soluble carbodiimides (WSCDI) like 1-ethyl-3-(3-dimethylaminopropyl)carbodiimide (EDC), optionally as salt like as hydrochloride salt.
Other coupling techniques use pre-formed active esters, such as N-hydroxysuccinimide (HOSu) and p-nitrophenol (HONp) esters, pre-formed symmetrical anhydrides, non-symmetrical anhydrides such as N-carboxyanhydrides (NCAs) and acid halides, such as acyl fluorides or acyl chlorides.
Preferred coupling reagents are carbodiimide coupling reagents and phoshonium coupling reagents, most preferred coupling reagents are selected from group consisting of PyBOP, DCC, DIC and EDC;
more preferably, coupling reagent is PyBOP or DIC.
EDC is preferably used as a salt, more preferably as EDC.HCl.
Preferably, the molar amount of coupling reagent is from 1 to 10 times, more preferably from 1 to 5 times, of the molar loading capacity of the resin, or of the molar amount of the fragment to be coupled in LPPS respectively.

More preferably, the molar amount of coupling reagent is from 1 to 10 times, more preferably from 1 to 5 times, of the molar amount of the substrate, such as the AA, to be coupled.

COUPL can be done in the presence of a base, preferably a tertiary amine base, which both deprotonates the COOH residue of the carboxylic component and neutralizes any counterion of the NH₂ residue of the amino component in COUPL, and thus facilitates the coupling reaction.

Suitable bases are for example trialkylamines, like N,N-diisopropylethylamine (DIPEA) or triethylamine (TEA);
N,N-dialkylanilines, like N,N-diethylaniline;
2,4,6-trialkylpyridines, like 2,4,6-trimethylpyridine;
N,N-dialkylaminopyridines, like N,N-4-dimethylaminopyridine; and
N-alkylmorpholines, like N-methylmorpholine.
Preferably, COUPL is done in the presence of DIPEA as a base.
Preferably, the molar amount of base is from 0.01 to 20 times, more preferably from 0.02 to 10 times, of the molar loading capacity of the resin.
More preferably, the molar amount of base is from 0.01 to 20 times, more preferably from 0.02 to 10 times, of the molar amount of the substrate, such as the AA, to be coupled.

COUPL can be done in the presence of an auxiliary nucleophile as additive due to their positive effect in suppressing undesired side reactions. Any known auxiliary nucleophile may be used.
Examples of suitable auxiliary nucleophiles are ethyl (hydroxyimino)cyanoacetate, 1-hydroxybenzotriazole (HOBt), N-hydroxysuccinimide (HOSu), N-hydroxy-3,4-dihydro-4-oxo-1,2,3-benzotriazine (HOOBt) and 1-hydroxy-7-azabenzotriazole (HOAt).
Preferably, HOBt or HOAt are used as auxiliary nucleophile.
Preferably, the molar amount of auxiliary nucleophile is from 0.5 to 8 times of the molar amount of the amino residue that is to be coupled.

Especially, COUPL is done using DIC/OxymaPure, DCC/HOBt, EDC/HOBt, PyBOP/HOBt or EDC/HOAt.

Preferably, COUPL can be done in any inert solvent which can dissolve the reactants. Preferred solvents for COUPL are water-miscible solvents like dimethyl sulfoxide (DMSO), dioxane, tetrahydrofurans such as tetrahydrofurane (THF) or methyltetrahydrofurane (Methyl-THF), 1-methyl-2-pyrrolidone (NMP), N,N-dimethylformamide (DMF), N,N-dimethylacetamide (DMA), or any mixture thereof; and non water-miscible solvents like dichloromethane (DCM), ethyl acetate or any mixture thereof; and any suitable mixture between water-miscible and non water-miscible solvents, including mixtures with water.
More preferably, COUPL is done in a solvent selected from the group consisting of DMSO, dichloromethane, Me-THF, DMF, NMP, and mixtures thereof.

Preferably, the substrate in COUPL, for example any AA, that is reacted with the respective functional residue connected to the resin in SPPS, is preferably used in excess with regard to the molar loading capacity of the resin;
more preferably, it is used in an molar amount of from 1 to 10 times, more preferably from 1 to 8 times, based on the molar loading capacity of the resin.

Preferably, COUPL is done at atmospheric pressure.
Preferably, the reaction temperature of COUPL is from -20 to 70°C; more preferably from -15 and 40°C, even more preferably from -15 to 35°C.
Preferably, the reaction time of COUPL is from 30 min to 48 h, more preferably from 30 min to 24 h, even more preferably from 30 min to 12 h, especially from 30 min to 6 h.
Preferably, the progress of COUPL is monitored by HPLC and thereby the necessary reaction time is determined.

Fmoc cleavage is known to the skilled person, it is done with a base, preferably the base is a secondary amine, more preferably piperidine.
The base is used in excess with regard to the molar loading capacity of the resin.
Preferably, DMF is used as solvent in the Fmoc cleavage reaction.
Preferably, the Fmoc cleavage is done at atmospheric pressure.
Preferably, the reaction temperature of the Fmoc cleavage is from -20 to 70°C; more preferably from -15 and 40°C, even more preferably from -15 to 35°C.
Preferably, the reaction time of the Fmoc cleavage is from 1 min to 12 h, more preferably from 1 min to 6 h, even more preferably from 1 min to 1 h.

Liraglutide can be isolated, preferably after a global deprotection, according to standard methods known to the skilled such as precipitation, preferably with ether, centrifugation, filtration etc.

Liraglutide can be purified according to standard methods known to the skilled person, such as HPLC.

### Examples

### AA

The SPPS was done with the following AA, if not stated otherwise:
Boc-His(Trt)-OH
Fmoc-Ala-OH
Fmoc-Glu(OtBu)-OH
Fmoc-Gly-OH
Fmoc-Thr(tBu)-OH
Fmoc-Phe-OH
Fmoc-Ser(tBu)-OH
Fmoc-Asp(OtBu)-OH
Fmoc-Tyr(tBu)-OH
Fmoc-Val-Ser[Psi(Me,Me)Pro]-OH optionally for position (10) and (11)
Fmoc-Leu-OH
Fmoc-Gln(Trt)-OH
Fmoc-Lys(Alloc)-OH
Fmoc-Ile-OH
Fmoc-Trp(Boc)-OH
Fmoc-Val-OH
Fmoc-Arg(Pbf)-OH

SPPS was done manually without an automatic synthesizer.

**Palm-Glu(OSu)-OtBu,** that is compound of formula (10), was purchased from IRIS Biotech; 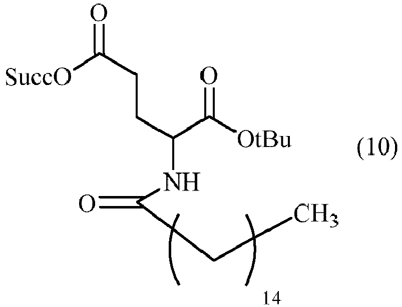 with SuccO being residue of formula (SuccO); 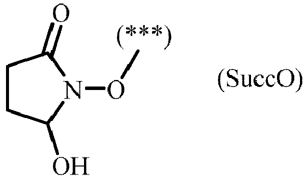 wherein the (***) denotes the covalent bond to the CO residue in compound of formula (10).

Compound of formula (BAL-1) was purchased from Novabiochem.

### Ninhydrin Test, also called Kaiser Test

The test is known to the skilled person, see Kaiser E. et al., Analytical Biochemistry 1970, 34, 595-598.
Solution 1: Dissolve 5 g ninhydrin in 100 mL ethanol.
Solution 2: Dissolve 40 g phenol in 10 mL ethanol.
Solution 3: Add 2 mL of a 0.001 M aq. KCN solution to 98 mL pyridine.

### Procedure:

1. Wash resin with DCM (2 times).
2. Sample a few beads of resin in an Eppendorf tube.
3. Add one to two drops of each of the three solutions 1 to 3.
4. Mix well and heat to 120 °C for 3 to 5 min.
5. The presences of free resin-bound amines are indicated by blue resin beads.

### Aldehyde Test

Jesus Vazquez et al., Tetrahedron Letters 2001, 42(38), 6691-6693

### Washing with a solvent / treating the resin with reagent

In the following example washing with a solvent such as DMF or DCM was done be filtering the resin, suspending and stirring the resin in the solvent for a certain time and filtering again. All washings were carried out with 10 mL (ca. 15 mL/g of resin) of DMF until Ala⁽¹⁸⁾, then with 15 mL.
Stated in the examples are the number of repetitions of this washing cycle together with the time for stirring. Also in case of treating the resin with a reagent that is dissolved in a solvent this description of the number of repetitions of this treatment together with the time for stirring can be used in the example, if not otherwise stated.
The introduction of the amino acids was done with 5 mL of DMF, if not otherwise stated.

### Solvent for coupling

Solvent for coupling was used in the amount of 20 ml/g, if not otherwise stated.

### Yield

The yield was calculated on the basis of the synthesis scale with respect to resin loading capacity f and resin weight, if not otherwise stated.

### Method for determination of purity

Peptide-resin (100 mg) is treated with ca. 2 ml of TFA : H2O: TIS in the volume ratio 94:3:3 and stirring for 1 h at RT. The suspension is filtered. The deprotected peptide is precipitated by addition of diisopropyl ether (20 ml) to the filtrate. The purity of crude deprotected peptide is checked by HPLC:

### Determination of purity of crude liraglutide:

- Column with C18 stationary phase
- Mobile phase A: 0.1% (v/v) TFA in water
- Mobile phase B: 0.1% (v/v) TFA in acetonitrile
- Gradient: 5% (v/v) B to 100% (v/v) B in 11 min
- Temperature: RT
- Flow rate : 1 ml/min
- Detection: 254 nm

### Fmoc determination, also used for determination of resin loading:

An analytical method based on the UV absorption of dibenzofulvene after cleavage of Fmoc on an aliquot of peptide-resin, the method is known to the skilled person. It can be done as follows:
An aliquot of loaded resin (ca. 100 mg) is washed with DMF (3 times with 5 ml) and DCM (3 times with 5 ml). It is then dried under vacuum.
10 mg of dried resin is added to a 2 mL polypropylene tube, 0.8 mL of DMF is added and the resin is allowed to swell for 10 minutes. Then 0.2 mL of piperidine is added, then the mixture is shaken for 20 min. Then 10 microL of the supernatant is pipetted into a quartz cuvette and is diluted with 0.990 mL of DMF. The absorbance at lamda = 301 nm is determined. The piperidine-dibenzofulvene adduct that is formed upon Fmoc deprotection has a molar extinction coefficient of epsilon = 7800 L x mol-1 x cm-1; thus, the loading value is determined using Beer-Lambert law.

### Example 1: Synthesis of Liraglutide

### Synthesis of Liraglutide on ChemMatrix® resin was done according to Scheme 1:

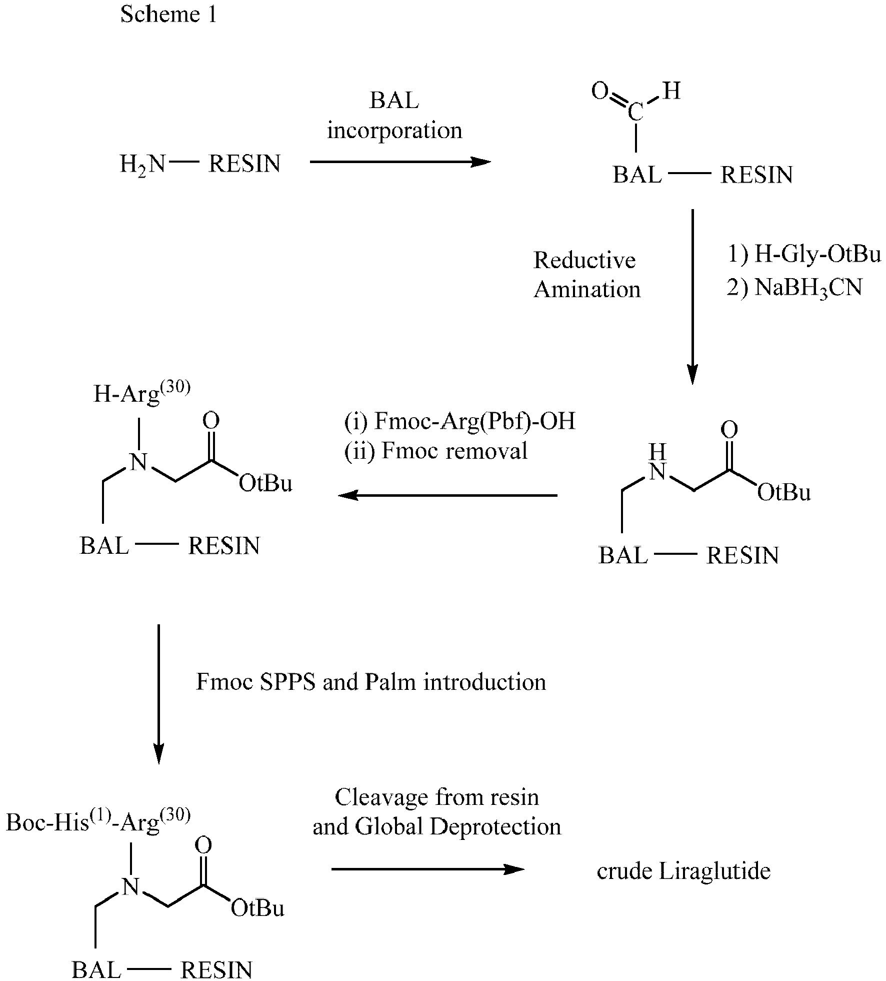

### BAL incorporation

The synthesis was performed using Aminomethyl ChemMatrix® (purchased from ChemMatrix® Innovation) resin (f = 0.58 mmol/g). The resin (0.6 g, 1 eq) was washed with DCM (3 times). After that, BAL linker (6 eq, 560 mg) was coupled onto the resin with DIC/OxymaPure® (6 eq, 263 microL/296 mg) in DMF.

The resin was then stirred for 4 hours. Then the resin was filtered and was washed with DMF (5 times for 1 min each) and DCM (5 times for 1 min each).
Ninhydrin test was performed (negative) and aldehyde test (positive) confirmed that the BAL linker was effectively coupled.

### Reductive Amination

The resin was suspended in DMF and introduction of H-Gly-OtBu was carried out by on-resin reductive amination using the free amine H-Gly-OtBu (10 eq, 579 mg) in form of a solution in 1% (v/v) AcOH in DMF (ca. 100 microL of AcOH per g of resin) for 30 min and then adding a solution of NaBH₃CN (10 eq, 218 mg) in 11 ml of MeOH overnight. After that, the resin was washed with DMF (5 times for 1 min each) and DCM (5 times for 1 min each). The reaction was monitored by aldehyde test (negative).

### Arg acylation

Fmoc-Arg(Pbf)-OH (3 eq, 677mg) was then coupled using DIC/OxymaPure® (3 eq 131 microL/148 mg) in 45 ml of DMF as coupling reagents for 90 min. To avoid amino acid deletion, the coupling of the Fmoc-Arg(Pbf)-OH was repeated under the same conditions. After that, Fmoc group was removed [by (i) washing the resin with DMF (5 times for 1 min each); (ii) treatment of the resin in piperidine : DMF (2 : 8 v/v, 5 ml/g resin) (1 time for 1 min and 2 times for 10 min each); (iii) washing with DMF (5 times for 1 min each)]. Ninhydrin test was performed with a positive result.

### Coupling of the remaining AA

Coupling of the remaining AA was carried out by adding the reagents AA : DIC : OxymaPure® in the ratio 3 eq :3 eq :3 eq and then stirring for 90 min.
The amino acids Val(¹⁰) and Ser⁽¹¹⁾ were coupled in form of the pseudo-Proline Fmoc-Val-Ser(psi^{Me,Me}pro)-OH.

### Removal of Fmoc

Removal of Fmoc was done by treatment with piperidine : DMF (2 : 8 v/v, 5 ml/g resin) for 1 time for 1 min and 1 time for 15 min.

### Palm introduction

During the synthesis, Lys was introduced as Fmoc-Lys(Alloc)-OH. After the coupling of Ala⁽¹⁸⁾ and before the removal of its Fmoc protecting group, the Alloc protecting group of the side chain of Lys⁽²⁰⁾ was removed with Pd(PPh₃)₄/PhSiH [by (i) washing the resin with DCM (5 times for 1 min); (ii) treatment of the resin at RT with Pd(PPh₃)₄/PhSiH in the ratio 0.1 eq : 10 eq (2 times for 10 min in DCM); (iii) washing with DCM (5 times for 1 min)]. Then, the resin was treated with Palm-Glu(OSu)-OtBu (3 eq) and DIPEA (6 eq, 265 microL) in DCM for 16 h.
After Palm introduction the coupling was continued.

### Cleavage from resin & Global Deprotection & Isolation of crude liraglutide

After peptide backbone was finished, the peptidyl-resin was washed with DCM (5 time for 1min) and MeOH (5 times for 1 min).

Cleavages were done in batches of 0.2 to 0.5 g portions. The cleavage was done with 10 mL of a cocktail [TFA : H2O: -TIS (94 : 3 : 3 volume)]/g of resin. After filtration the resin was washed twice with TFA : H2O: TIS (94 : 3 : 3 volume) (5 mL) and the TFA solution was concentrated to one third of its volume and then added onto tert-buthylmethyl ether (5 times of the volume of the cleavage solution after concentration) and centrifugated. After decantation, the solid was washed twice with the same amount of tert-buthylmethyl ether and again centrifugated and decanted. The solid was lyophilized.

Yield of crude was 1.06 g, purity of crude was 62 %.

### Purifcation

The following purification method is a standard purification method which is not optimized for liraglutide and for yield.
The crude unprotected Liraglutide was purified on XSelect® CSH C18 semi-preparative HPLC column (Waters).
Mobile phase A: 0.1% (v/v) TFA in water
Mobile phase B: 0.1% TFA (v/v) in acetonitrile

### Gradient:

| **t** | **A** | **B** |
|---|---|---|
| **[min]** | [%] | [%] |
| **0** | 95 | 5 |
| **3** | 95 | 5 |
| **4** | 51 | 49 |
| **9** | 48.5 | 51.5 |
| **10** | 0 | 100 |
| **12** | 0 | 100 |
| **12.5** | 95 | 5 |

Liraglutide elutes at about minute 7 of the gradient.
Purity: >99%
Purification yield: ca. 10%

### Example 2

Example 1 was repeated with the difference that the amino acids Val(¹⁰) and Ser⁽¹¹⁾ were coupled in form of Fmoc-Ser(tBu)-OH and of Fmoc-Val-OH respectively, that is they were not used on form of a pseudo-Proline.

Purity of crude liraglutide obtained without using pseudo-Proline was ca. 40%.

### Example 3

Example 1 was repeated with the difference that the introduction of the Palmitoyl-Glu-OtBu residue was not done after Ala(18), but after the final coupling of Boc-His(Trt)-OH using the same procedure.
This means that after the final coupling of Boc-His(Trt)-OH the Alloc on Lys⁽²⁰⁾ side chain was the selectively cleaved and then the Palmitoyl-Glu-OtBu residue was introduced on resin.

Purity of crude liraglutide was ca. 60%

## Claims

1. Method for the preparation of liraglutide with SPPS using a backbone amide linker BAL for connecting the C-terminal Gly⁽³¹⁾ with the resin during SPPS;
BAL is compound of formula (BAL); 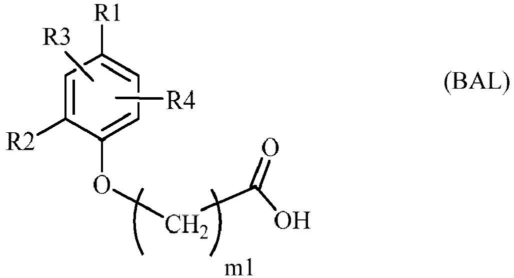 wherein
one of the substituents R1 and R2 is H and the other one is C(O)H;
ml is 1, 2, 3, 4, 5, 6, 7, 8, 9 or 10;
R3 and R4 are identical or different and independently from each other H or methoxy.

2. Method according to claim 1, wherein
ml is 2, 3, 4, 5, 6 or 7.

3. Method according to claim 1 or 2, wherein
R3 and R4 are on the 3 and on the 5 position of the phenoxy ring with respect to the position of the carboxylic acid residue on position 1 of the phenoxy ring.

4. Method according to one or more of claims 1 to 3, wherein
R2 is H and R1 is C(O)H.

5. Method according to one or more of claims 1 to 4, wherein
the SPPS is done on an amino functionalized resin AMFUNRESIN.

6. Method according to one or more of claims 1 to 5, wherein
AMFUNRESIN is selected from the group consisting of aminomethyl ChemMatrix® resin, aminomethyl polystyrene resin, Rink amide resin, Sieber resin, 4-methyl-benzhydrylamine resin (MBHA resin), poly Lys resin, and benzhydrylamine resin (BHA resin).

7. Method according to one or more of claims 1 to 6, wherein
BAL is covalently connected with the resin by an amide bond between the COOH of BAL and the NH₂ of AMFUNRESIN.

8. Method according to one or more of claims 1 to 7, wherein
Gly⁽³¹⁾ is covalently connected with BAL by a bond between the alpha NH₂ of Gly⁽³¹⁾ and the C atom of the C(O)H residue of BAL.

9. Method according to one or more of claims 1 to 8, wherein
liraglutide is prepared by SPPS by firstly covalently connecting the BAL with AMFUNRESIN, thereafter the alpha NH₂ of Gly⁽³¹⁾ is covalently connected with the C(O)H residue of BAL, then the other AA are coupled by SPPS onto the Gly⁽³¹⁾ and then onto the growing peptide chain respectively.

10. Method according to claim 8 or 9, wherein
the covalently connecting of the alpha NH₂ of Gly⁽³¹⁾ with the C(O)H residue of BAL is done with a reductive amination reaction.

11. Method according to one or more of claims 1 to 10, wherein
the method comprises two steps, a step STEP 1 and a step STEP2;
STEP 1 comprises the SPPS using a backbone amide linker BAL;
STEP 1 provides liraglutide connected to the resin of the SPPS;
STEP2 comprises cleavage of liraglutide from the resin.

12. Method according to one or more of claims 1 to 11, wherein
those AA, that can be side chain protected, are used in a side chain protected form in the SPPS.

13. Method according to claim 12, wherein
the side chain protection is realized in form of side chain protection groups which are not cleavable under weakly acidic conditions.

14. Method according to claim 12 or 13, wherein
side chain protection of Cys is Trt or Acm;
side chain protection of Asn and Gln is Trt;
side chain protection of His is Trt or Dnp;
side chain protection of Glu and Asp is OtBu;
side chain protection of Thr, Ser and Tyr is tBu;
side chain protection of Trp is Boc;
side chain protection of Arg is Pbf, Pmc, Mtr or MIS;
side chain protection of Lys is Alloc, Z or Boc.

15. Method according to one or more of claims 1 to 14, wherein
the Val(¹⁰) and the Ser⁽¹¹⁾ are used in the SPPS in form of pseudoproline Fmoc-Val-Ser[Psi^{(Me,Me)}Pro]-OH.

16. Method according to one or more of claims 1 to 15, wherein
the SPPS uses Fmoc/tBu strategy.

17. Method according to one or more of claims 1 to 16, wherein
the Palmitoyl-Glu-OtBu residue on the N⁶ of the Lys⁽²⁰⁾ is the residue of formula (PALGLU), 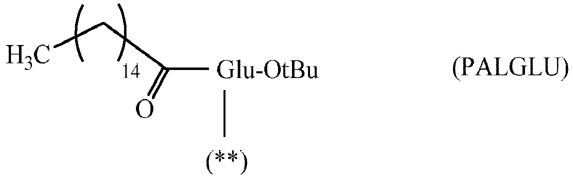
with the (**) in formula (PALGLU) denoting the covalent bond between the COOH of the side chain of the Glu and the N⁶ of the Lys⁽²⁰⁾,
and the Palmitoyl-Glu-OtBu residue is covalently bonded to the Lys⁽²⁰⁾ in a reaction REACPALGLU, wherein the NH₂ of the side chain of the Lys is reacted with a precursor of the residue of formula (PALGLU).

18. Method according to claim 17, wherein
REACPALGLU is done before the peptide is cleaved from the resin.

19. Method according to claim 17 or 18, wherein
the precursor of the residue of formula (PALGLU) is is compound of formula (10); 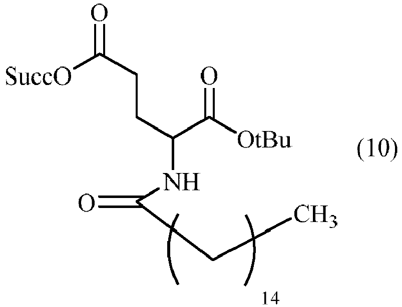 with SuccO being residue of formula (SuccO); 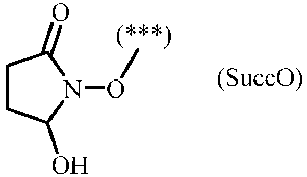 wherein the (***) denotes the covalent bond to the CO residue in compound of formula (10).

20. Method according to one or more of claims 17 to 19, wherein
REACPALGLU is done after the coupling of Ala⁽¹⁹⁾ or after the coupling of Ala⁽¹⁸⁾, and before removal of the protecting group of the alpha NH₂ of Ala⁽¹⁹⁾ or of Ala⁽¹⁸⁾ respectively.
